# FASCICULE DE BREVET EUROPEEN

(11) **EP 0 676 216 B1**
(45) Date de publication et mention de la délivrance du brevet: **28.08.2002**
(21) Numéro de dépôt: 95400755.5
(22) Date de dépôt: 05.04.1995
(51) Int. Cl.: A61N 1/368

(54) **Stimulateur cardiaque auriculaire double du type triple chambre**
Doppeltvorhofherzschrittmacher mit Steuerung als Dreikammerherzschrittmacher
Double atrial pacemaker with triple-chamber control

(30) Priorité: 05.04.1994 FR 9403988
(43) Date de publication de la demande: 11.10.1995
(73) Titulaire: ELA MEDICAL (Société anonyme), F-92541 Montrouge (FR)
(72) Inventeur: Limousin, Marcel, F-92120 Montrouge (FR)
(74) Mandataire: Dupuis-Latour, Dominique

(56) Documents cités:
- WO-A-92/09331
- WO-A-92/14511
- US-A- 5 107 850

## Description

L'invention concerne les stimulateurs cardiaques du type "triple chambre", c'est-à-dire ceux permettant une stimulation du ventricule droit et de chacune des deux oreillettes, droite et gauche.

Plus précisément, ces stimulateurs comportent, outre une sonde ventriculaire (généralement bipolaire), deux sondes auriculaires implantées sur chacune des deux oreillettes et reliées à une borne correspondante, unique, du stimulateur par l'intermédiaire d'un connecteur en Y. La sonde auriculaire est ainsi une sonde double mais, à la différence des électrodes d'une sonde "bipolaire" classique, où les deux extrémités distale et proximale ne sont éloignées que de quelques millimètres, les deux extrémités des sondes sont relativement éloignées, d'une valeur typique de l'ordre de 5 cm.

Ces stimulateurs cardiaques triple chambre sont utilisés de façon relativement satisfaisante depuis quelques années dans les indications de patients présentant un dysfonctionnement sinusal du type "bloc inter-auriculaire" conduisant à une propagation déficiente (insuffisante ou trop longue) de l'oreillette droite à l'oreillette gauche. Ainsi, si l'on ne stimulait que l'une des oreillettes (situation classique des stimulateurs "double chambre"), l'oreillette gauche, non stimulée, recevrait l'onde de dépolarisation provenant de l'oreillette droite, stimulée, avec un délai excessivement long, quelquefois plus long que le délai auriculo-ventriculaire. Un tel phénomène peut conduire à une contraction des ventricules survenant avant vidange de l'oreillette gauche, donc avant fermeture de la valvule mitrale, produisant ainsi un contre-flux sanguin du ventricule vers l'oreillette et une diminution de l'efficacité hémodynamique. De plus, la désynchronisation électrique des deux oreillettes favorise l'apparition de tachyarythmies. En outre, le délai inter-auriculaire augmentant avec l'effort, l'accroissement de l'activité physiologique du patient favorise le risque d'apparition d'un tel syndrome. Les stimulateurs "triple chambre", en stimulant l'une et l'autre oreillette de façon simultanée, évitent l'apparition ou la persistance de ce type de phénomènes.

Toutefois, les études cliniques ont révélé, pour certains patients, l'apparition de tachyarythmies auriculaires (TA) en dépit d'une telle stimulation systématique des deux oreillettes. Ces TA, dont l'origine n'avait jusqu'à présent pas pu être décelée, nécessitent un traitement médicamenteux des patients (par exemple une thérapie par des bêtabloquants), mais avec un certain taux d'échec résiduel, un tel traitement n'étant pas suffisant, chez certains patients, pour empêcher l'apparition de telles TA récurrentes. En outre, le traitement médicamenteux des TA peut présenter chez certains patients des contre-indications absolues qui interdisent sa mise en oeuvre.

L'un des buts de l'invention est de proposer un stimulateur cardiaque "triple chambre" permettant de vaincre la quasi-totalité des TA résiduelles d'origine inconnue associées à ce type d'appareil, en s'affranchissant en outre de tout traitement médicamenteux, et donc des difficultés liées aux contre-indications et aux effets secondaires des agents anti-arythmiques qu'il était jusqu'à présent nécessaire d'utiliser, pour certains patients, en supplément de la stimulation triple chambre.

L'invention est essentiellement basée sur la découverte de la cause probable des TA persistantes associées aux stimulateurs triple chambre qui, comme on l'expliquera plus en détail par la suite, sont très vraisemblablement des tachycardies ré-entrantes électroniques ("TRE" ou "PMT", *Pacemaker-Mediated Tachyarrhythmia*) engendrées par une confusion intervenant, dans certaines situations, au niveau des circuits de détection auriculaire du stimulateur, entre les signaux auriculaires, qui peuvent se présenter sous la forme de doublets si le temps de propagation inter-oreillettes est important, et des fronts d'onde ventriculaire provenant d'un foyer ectopique, notamment si ce foyer est situé près de l'oreillette gauche. Il peut résulter alors de cette confusion une stimulation à contretemps susceptible d'induire une TA récurrente. L'objet de l'invention est ainsi de procéder à une discrimination permettant d'éviter la confusion entre signaux d'origine ventriculaire et d'origine auriculaire, et d'éviter ainsi le déclenchement de TRE à l'origine des TA persistantes d'origine jusqu'à présent inconnue associées à la stimulation triple chambre.

L'invention s'applique, comme on vient de le dire, à un stimulateur cardiaque auriculaire double du type triple chambre, c'est-à-dire comprenant les caractéristiques visées au préambule de la revendication 1.

Selon l'invention, il comprend les moyens énoncés par la partie caractérisante de la revendication 1.

Les sous-revendications 2 et 3 visent des mises en oeuvre avantageuses.

D'autres caractéristiques et avantages de l'invention apparaîtront à la lecture de la description ci-dessous, faite en référence aux dessins annexés.

La figure 1 est une vue schématique des différents organes d'un stimulateur triple chambre et de l'implantation de ses sondes sur le myocarde.

La figure 2 illustre la succession dans le temps des divers fronts d'onde de dépolarisation recueillis par le circuit de détection auriculaire du stimulateur, permettant d'expliciter le principe de l'invention.

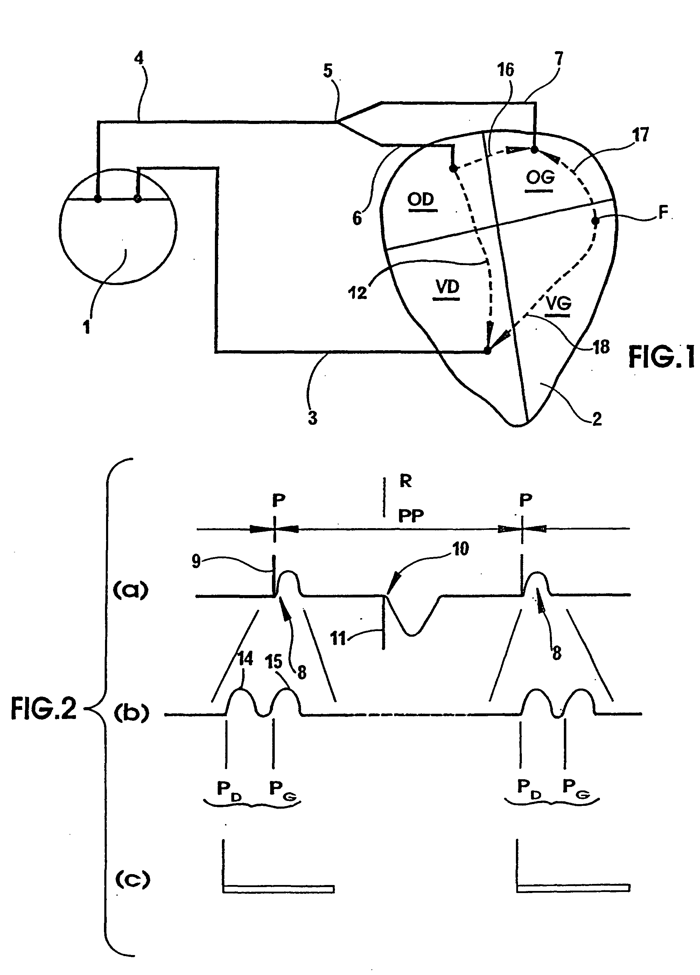

Sur la figure 1, la référence 1 désigne le stimulateur cardiaque, qui est d'un type classique DDD ou DDT, c'est-à-dire un stimulateur double bipolaire (détection des signaux auriculaires et ventriculaires et stimulation auriculaire et ventriculaire), fonctionnant en mode déclenché, c'est-à-dire produisant une stimulation dès le recueil d'un signal sur la sonde correspondante ou, de façon imposée, en l'absence de recueil d'un signal après écoulement d'un laps de temps donné.

Le stimulateur 1 est relié au myocarde 2 par une configuration du type triple chambre, c'est-à-dire avec une sonde ventriculaire 3 reliée au ventricule droit VD et une sonde auriculaire 4 reliée à chacune des deux oreillettes, droite OD et gauche OG par l'intermédiaire d'une bifurcation en Y, référencée 5, et de deux branches de sondes respectives 6 et 7. Du fait de ce montage en Y, le circuit de détection/ stimulation auriculaire du stimulateur relié à la sonde 4 (en entrée pour la détection et en sortie pour la stimulation) détecte tout signal recueilli par l'une quelconque des sondes 6 ou 7 indifféremment et, inversement, stimule simultanément et de façon identique les deux oreillettes.

Le signal recueilli par ce circuit auriculaire se présente généralement de la manière illustrée figure 2a, et comprend une succession d'ondes auriculaires (ondes P) telles que celle référencée 8, l'apparition d'une telle onde déclenchant immédiatement une stimulation auriculaire, correspondant au pic 9. Le circuit recueille ensuite l'onde ventriculaire 10 associée au pic 11 en cas de stimulation produite par la sonde 3. Le décalage temporel entre onde P et onde R correspond au délai atrio-ventriculaire (schématisé par la flèche 12 sur la figure 1). Bien entendu, en cas de bloc atrio-ventriculaire ou anomalie du même type, le circuit ventriculaire du stimulateur déclenche automatiquement la stimulation si aucune onde ventriculaire n'est détectée au bout d'un laps de temps prédéterminé.

Dans le cas particulier de la stimulation triple chambre, le front de dépolarisation auriculaire 8 peut se présenter, en l'absence de stimulation, de la manière illustrée à plus grande échelle figure 2b, c'est-à-dire sous la forme d'un doublet 14, 15, l'onde 14 correspondant à l'onde P de l'oreillette droite et l'onde 15, à l'onde P de l'oreillette gauche. L'espacement entre les deux ondes 14 et 15 dépend du délai inter-auriculaire (schématisé en 16 sur la figure 1), qui est variable selon les sujets et qui, chez un même sujet, augmente avec l'effort.

Par ailleurs, outre l'onde de dépolarisation ventriculaire, qui a son origine au droit de l'extrémité de la sonde 3, il peut apparaître un foyer ectopique d'excitation, pour lequel l'origine de l'onde ne se trouve pas à l'endroit où se trouvent les sondes, c'est-à-dire approximativement à l'extrémité des sondes 3, 6, mais peut, dans certains cas, se situer en un point du ventricule gauche relativement proche de l'oreillette gauche, par exemple comme illustré en F sur la figure 1. Compte tenu de cette proximité, le délai de propagation depuis le foyer F jusqu'à l'extrémité de la sonde auriculaire gauche 7, délai schématisé en 17 sur la figure 1, peut être d'une valeur proche, ou même plus courte, que le délai inter-auriculaire 16. Le front d'onde de dépolarisation correspondant d'origine ectopique risque donc d'interférer avec le front d'onde d'origine auriculaire et être interprété ― à tort ― par le stimulateur comme un signal auriculaire subséquent, déclenchant ainsi à contretemps une stimulation auriculaire (la stimulation est synchrone de la détection, puisque l'on est en mode DDT). Une telle stimulation prématurée et à contretemps des oreillettes induit une tachycardie du fait de la transmission auriculo-ventriculaire 12 et donc une contraction anticipée du ventricule. La reproduction à l'identique de ce mécanisme conduit à une tachycardie induite par le stimulateur.

L'invention résulte de la découverte de ce phénomène de tachycardie ré-entrante électronique propre aux stimulateurs triple chambre, qui semble très vraisemblablement être à l'origine des TA récurrentes inexpliquées rencontrées jusqu'à présent avec ce type d'appareil.

Pour empêcher ce phénomène, l'invention propose d'opérer une discrimination des signaux reçus par le circuit de détection auriculaire, et de n'opérer la stimulation auriculaire qu'à bon escient, en éliminant l'incidence des signaux issus de foyers ectopiques ventriculaires d'excitation.

Pour opérer la discrimination, selon une première forme de mise en oeuvre de l'invention, on mesure de façon continue (par le microprocesseur du stimulateur ou par des circuits à compteur) l'intervalle PP représenté figure 2a, c'est-à-dire l'intervalle entre deux signaux auriculaires successifs reçus, cette valeur étant mise à jour à chaque nouveau signal reçu en entrée du circuit de détection auriculaire.

On évalue alors, à chaque nouvelle mesure, la diminution ou le taux de diminution de cet intervalle de temps, c'est-à-dire la différence (absolue ou relative, respectivement) avec la valeur mesurée précédente et on compare cette diminution ou ce taux de diminution à une valeur limite de consigne prédéterminée, par exemple une diminution maximale de 125 ms ou un taux de diminution maximal de 25 %.

Si cette valeur n'est pas franchie, on considère que la situation est normale et on procède alors immédiatement à la stimulation auriculaire.

Dans le cas contraire, on ouvre une "fenêtre d'écoute" sur la sonde ventriculaire, par exemple de 31 ou 50 ms, c'est-à-dire que l'on surveille l'apparition éventuelle d'un signal en entrée du circuit de détection ventriculaire. Si un signal est reçu dans les limites de cette fenêtre, ceci signifie que le signal reçu en dernier lieu sur la sonde auriculaire était en fait issu d'une dépolarisation ectopique ventriculaire (correspondant au trajet de propagation schématisé en 18 sur la figure 1) et dans ce cas on ne procède à aucune stimulation auriculaire, afin d'éviter l'apparition d'une TRE résultante du phénomène explicité plus haut. Si, en revanche, aucun signal n'est reçu pendant la durée de cette fenêtre d'écoute, on considère que le signal reçu en dernier lieu sur la sonde auriculaire était effectivement un signal d'origine auriculaire et que l'accélération constatée était en fait une accélération physiologique de l'oreillette et l'on stimule l'oreillette à la fin de la fenêtre d'écoute (afin de conserver un délai minimal). En outre, le délai auriculo-ventriculaire déclenché sur cette dernière détection auriculaire pourra être allongé pour garder au moins une durée correspondant au cycle précédent.

Une autre manière de procéder à la détermination du caractère prématuré ou non de l'onde P consiste, selon une seconde forme de mise en oeuvre de l'invention, à se caler sur les événements ventriculaires. À cet effet, après chaque événement ventriculaire (détection ou stimulation) on déclenche une période de suspicion d'extra-systole ventriculaire correspondant à une fraction de l'intervalle P-P précédent ou de l'intervalle R-R précédent, cette fraction pouvant être définie en valeur relative (*x* % de l'intervalle précédent) ou en valeur absolue (intervalle précédent moins *x* millisecondes). Si l'on détecte durant cette période un signal en entrée du circuit de détection auriculaire, on est en droit de soupçonner la survenance d'une extra-systole ventriculaire et on ouvre alors, comme dans le mode de mise en oeuvre précédent, une fenêtre d'écoute sur la sonde ventriculaire. La suite des étapes se déroule de la même façon qu'exposé précédemment.

## Revendications

1. Un stimulateur cardiaque auriculaire double du type triple chambre (1), comportant une sonde auriculaire droite (6) et une sonde auriculaire gauche (7) reliées ensemble à un seul et même circuit de détection/stimulation auriculaire du stimulateur, ainsi qu'une sonde ventriculaire (3) reliée à un circuit de détection/stimulation ventriculaire du stimulateur, stimulateur **caractérisé en ce qu'**il comprend :
― des moyens aptes à recevoir en entrée du circuit auriculaire et du circuit ventriculaire une succession de signaux de dépolarisation ;
― des moyens aptes à déterminer le caractère éventuellement prématuré du signal reçu en entrée du circuit auriculaire ;
― des moyens aptes à :
∗ en cas de prématurité avérée, examiner, pendant la durée d'une fenêtre d'écoute de durée prédéterminée, les signaux éventuellement reçus en entrée du circuit de détection ventriculaire et :
. en cas de réception de signal ventriculaire, inhiber toute stimulation auriculaire corrélative, et
. en cas d'absence de réception d'un signal ventriculaire, procéder à une stimulation auriculaire à la fin de la durée de la fenêtre d'écoute ; et
∗ dans le cas contraire, procéder à une stimulation auriculaire immédiate synchrone à la détection du signal de dépolarisation auriculaire.

2. Le stimulateur de la revendication 1, dans lequel lesdits moyens aptes à déterminer le caractère éventuellement prématuré du signal reçu en entrée du circuit auriculaire comprennent des moyens aptes à :
∗ mesurer l'intervalle de temps séparant deux signaux auriculaires successifs ;
∗ déterminer la diminution ou le taux de diminution de cet intervalle de temps ; et
∗ comparer cette diminution ou ce taux de diminution à une valeur limite de consigne prédéterminée, ladite prématurité étant avérée lorsque cette limite est dépassée.

3. Le stimulateur de la revendication 1, dans lequel lesdits moyens aptes à déterminer le caractère éventuellement prématuré du signal reçu en entrée du circuit auriculaire comprennent des moyens aptes à :
∗ mesurer l'intervalle de temps séparant deux signaux soit auriculaires soit ventriculaires successifs ;
∗ compter, à partir du dernier événement ventriculaire recueilli, une période correspondant à une fraction prédéterminée de cet intervalle de temps ; et
∗ analyser pendant cette période les signaux reçus en entrée du circuit auriculaire, ladite prématurité étant avérée lorsqu'un signal est recueilli.

## Patentansprüche

1. Doppelvorhof-Herzschrittmacher des Drei-Kammer-Typs (1) mit einer rechten Vorhofsonde (6) und einer linken Vorhofsonde (7), zusammen verbunden mit ein- und derselben Vorhof-Detektions/Stimulations-Schaltung des Schrittmachers, sowie einer Herzkammersonde (3), verbunden mit einer Herzkammer-Detektions/Stimulations-Schaltung des Schrittmachers,
wobei der Schrittmacher **dadurch gekennzeichnet ist, dass** er aufweist:
- Mittel, geeignet zum Empfangen am Eingang der Vorhof-Schaltung und der Herzkammer-Schaltung eine Folge von Depolarisationssignalen;
- Mittel, geeignet zum Bestimmen des eventuell vorzeitigen Charakters des am Eingang der Vorhof-Schaltung empfangenen Signals;
- Mittel, geeignet zum:
∗ im Fall der bewahrheiteten Vorzeitigkeit Prüfen, während der Dauer eines Hörfensters von vorbestimmter Dauer, der eventuell empfangenen Signale am Eingang der Herzkammer-Detektionsschaltung, und
. im Fall des Herzkammer-Signalempfangs Einstellen jeglicher korrelativen Vorhof-Stimulation, und
. im Fall der Abwesenheit des Empfangs eines Herzkammersignals Vornehmen einer Vorhof-Stimulation am Ende der Dauer des Hörfensters; und
∗ andernfalls Vornehmen einer sofortigen Vorhof-Stimulation synchron mit der Detektion des Vorhof-Depolarisationssignals.

2. Herzschrittmacher gemäß Anspruch 1, bei welchem die Mittel, geeignet zum Bestimmen des eventuell vorzeitigen Charakters des am Eingang der Vorhof-Schaltung empfangenen Signals, Mittel aufweisen, die geeignet sind zum:
∗ Messen des Zeitintervalls, welches zwei sukzessive Vorhofsignale trennt;
∗ Bestimmen der Verkleinerung oder des Maßes der Verkleinerung des Zeitintervalls; und
∗ Vergleichen der Verkleinerung oder des Maßes der Verkleinerung mit einem vorbestimmten Einstellgrenzwert, wobei die Vorzeitigkeit sich bewahrheitet, wenn die Grenze überschritten ist.

3. Schrittmacher gemäß Anspruch 1, bei welchem die Mittel, die geeignet sind zum Bestimmen des eventuell vorzeitigen Charakters des am Eingang der Vorhof-Schaltung empfangenen Signals, Mittel aufweisen, die geeignet sind zum:
∗ Messen des Zeitintervalls, welches zwei sukzessive Vorhof- oder Herzkammer-Signale trennt;
∗ Zählen, vom letzten aufgenommenen Herzkammer-Ereignis, einer Periode, die einem vorbestimmten Bruchteil des Zeitintervalls entspricht; und
∗ Analysieren während dieser Periode der am Eingang der Vorhof-Schaltung empfangenen Signale, der Vorzeitigkeit, welche sich bewahrheitet, wenn ein Signal empfangen wird.

## Claims

1. A double auricular heart stimulator (1) of the triple-chamber type, comprising a right atrium probe (6) and a left atrium probe (7) connected together to a single common auricular detection/stimulation circuit of the stimulator, and a ventricular probe (3) connected to a ventricular detection/stimulation circuit of the stimulator,
the stimulator being **characterized in that** it comprises:
· means suitable for receiving a succession of depolarization signals input from the auricular circuit and from the ventricular circuit;
· means suitable for determining the possibly premature nature of the signal received from the auricular circuit; and
· means suitable for:
· in the event of prematurity being confirmed, acting during the duration of a listening window of predetermined duration, to examine any signals received from the ventricular detection circuit; and
· if a ventricular signal is received, inhibiting any corresponding auricular stimulation; and
· if no ventricular signal is received, proceeding with auricular stimulation when the listening window expires; and
· otherwise proceeding with immediate auricular stimulation synchronously on detection of the auricular depolarization signal.

2. The stimulator claim 1, in which said means suitable for determining the possibly premature nature of the signal received from the auricular circuit comprise means suitable for:
· measuring the time interval between two successive auricular signals;
· determining any shortening or reduction ratio in said time interval; and
· comparing this shortening or reduction ratio with a predetermined reference limit value, said prematurity being confirmed when said limit is exceeded.

3. The stimulator of claim 1, in which said means suitable for determining the possibly premature nature of the signal received from the auricular circuit comprises means suitable for:
· measuring the time interval between two successive signals, either auricular signals or ventricular signals;
· starting from the most recently picked up ventricular event, measuring a period corresponding to a predetermined fraction of said time interval; and
· during said measured period, analyzing the signals received from the auricular circuit, said prematurity being confirmed when a signal is picked up.
